(19) Europäisches Patentamt
European Patent Office
Office européen des brevets

(11) Veröffentlichungsnummer : **0 140 833**
**B1**

(12) # EUROPÄISCHE PATENTSCHRIFT

(45) Veröffentlichungstag der Patentschrift : **02.03.88**

(51) Int. Cl.[4] : **C 07 D491/048**, B 41 M 5/12

(21) Anmeldenummer : **84810439.4**

(22) Anmeldetag : **10.09.84**

(54) **5-und 6-Azaphthalide, ihre Isomerengemische, Verfahren zu ihrer Herstellung und ihre Verwendung in druck- und wärmeempfindlichen Aufzeichnungsmaterialien.**

(30) Priorität : **15.09.83 CH 5030/83**

(43) Veröffentlichungstag der Anmeldung : **08.05.85 Patentblatt 85/19**

(45) Bekanntmachung des Hinweises auf die Patenterteilung : **02.03.88 Patentblatt 88/09**

(84) Benannte Vertragsstaaten : **BE CH DE FR GB IT LI**

(56) Entgegenhaltungen :
**EP-A- 0 082 822**
**AT-B- 354 475**
**DE-A- 2 842 263**
**DE-A- 3 116 815**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Klybeckstrasse 141**
**CH-4002 Basel (CH)**

(72) Erfinder : **Bedekovic, Davor, Dr.**
**Stefanstrasse 12**
**CH-4106 Therwil (CH)**
Erfinder : **Fletcher, Ian John, Dr.**
**Bürgenstal 27**
**CH-4312 Magden (CH)**

**EP 0 140 833 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft 5- und 6-Azaphthalide und deren Isomerengemische sowie Verfahren zu ihrer Herstellung und ihre Verwendung als Farbbildner in druckempfindlichen oder wärmeempfindlichen Aufzeichnungsmaterialien.

Die erfindungsgemässen Azaphthalide entsprechen der allgemeinen Formel

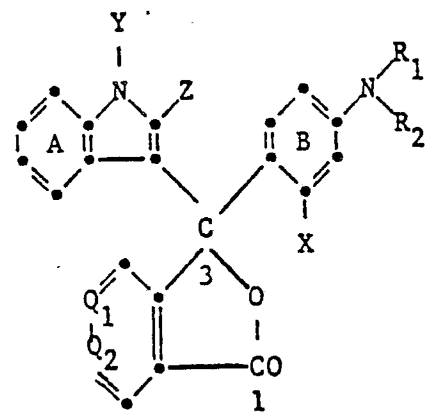

(1)

worin von $Q_1$ und $Q_2$ eines N und das andere CH,

Y Wasserstoff, unsubstituiertes oder durch Halogen, Hydroxyl, Cyano oder Niederalkoxy substituiertes Alkyl mit höchstens 12 Kohlenstoffatomen, Acyl mit 1 bis 12 Kohlenstoffatomen oder unsubstituiertes oder durch Halogen, Cyano, Niederalkyl oder Niederalkoxy substituiertes Benzyl,

Z Wasserstoff, Niederalkyl oder Phenyl,

$R_1$ und $R_2$, unabhängig voneinander, Wasserstoff, unsubstituiertes oder durch Halogen, Hydroxyl, Cyano oder Niederalkoxy substituiertes Alkyl mit höchstens 12 Kohlenstoffatomen, Cycloalkyl mit 5 bis 10 Kohlenstoffatomen oder unsubstituiertes oder durch Halogen, Cyano, Niederalkyl oder Niederalkoxy substituiertes Benzyl oder Phenyl oder

$R_1$ und $R_2$ zusammen mit dem sie verbindenden Stickstoffatom einen fünf- oder sechsgliedrigen, vorzugsweise gesättigten, heterocyclischen Rest,

X Wasserstoff, Halogen, Niederalkyl, $C_1$-$C_{12}$-Alkoxy, $C_1$-$C_{12}$-Acyloxy, Benzyl, Phenyl, Benzyloxy, Phenyloxy, durch Halogen, Cyano, Niederalkyl oder Niederalkoxy substituiertes Benzyl oder Benzyloxy, oder die Gruppe $—NT_1T_2$, $T_1$ und $T_2$, unabhängig voneinander je Wasserstoff, Niederalkyl, Cycloalkyl, unsubstituiertes oder durch Halogen, Cyano, Niederalkyl oder Niederalkoxy substituiertes Benzyl oder Acyl mit 1 bis 12 Kohlenstoffatomen und $T_1$ auch unsubstituiertes oder durch Halogen, Cyano, Niederalkyl oder Niederalkoxy substituiertes Phenyl bedeuten, und

worin der Ring A unsubstituiert oder durch Halogen, Cyano, Niederalkyl, Niederalkoxy, Niederalkoxycarbonyl, Amino, Mononiederalkylamino oder Diniederalkylamino substituiert ist und der Benzolkern B durch Halogen, Niederalkyl oder Niederalkoxy weiter substituiert sein kann.

Niederalkyl und Niederalkoxy stellen bei der Definition der Reste der Azaphthalide solche Gruppen oder Gruppenbestandteile dar, die 1 bis 5, insbesondere 1 bis 3 Kohlenstoffatome aufweisen, wie z. B. Methyl, Ethyl, n-Propyl, Isopropyl, n-Butyl, sek.-Butyl, Amyl oder Isoamyl bzw. Methoxy, Ethoxy, Isopropoxy, Isobutoxy oder tert. Butoxy.

Acyl ist besonders Formyl, Niederalkylcarbonyl, wie z. B. Acetyl oder Propionyl, oder Benzoyl. Weitere Acylreste können Niederalkylsulfonyl, wie z. B. Methylsulfonyl oder Ethylsulfonyl sowie Phenylsulfonyl sein.

Benzoyl und Phenylsulfonyl können durch Halogen, Methyl, Methoxy oder Ethoxy substituiert sein. Der Acyloxyrest in X ist beispielsweise Formyloxy, Niederalkylcarbonyloxy oder Benzoyloxy. Als $C_1$-$C_{12}$-Alkoxyrest kann X eine geradkettige oder verzweigte Gruppe sein, wie z. B. Methoxy, Ethoxy, Isopropoxy, tert. Butoxy, n-Hexyloxy, Octyloxy oder Dodecyloxy.

Stellen die Substituenten $R_1$, $R_2$ und Y Alkylgruppen dar, so können sie geradkettige oder verzweigte Alkylreste sein. Beispiele für solche Alkylreste sind Methyl, Ethyl, n-Propyl, Isopropyl, n-Butyl, sek.-Butyl, tert.-Butyl, Amyl, Isoamyl, n-Hexyl, 2-Ethyl-hexyl, n-Heptyl, n-Octyl, Isooctyl, n-Nonyl, Isononyl oder n-Dodecyl.

Sind die Alkylreste in $R_1$, $R_2$ und Y substituiert, so handelt es sich vor allem um Cyanoalkyl, Halogenalkyl, Hydroxyalkyl, Alkoxyalkyl jeweils vorzugsweise mit insgesamt 2 bis 4 Kohlenstoffatomen, wie z. B. β-Cyanoethyl, β-Chlorethyl, β-Hydroxyethyl, β-Methoxyethyl oder β-Ethoxyethyl.

Beispiele für Cycloalkyl in der Bedeutung der R- und T-Reste sind Cyclopentyl, Cycloheptyl oder vorzugsweise Cyclohexyl. Die Cycloalkylreste können einen oder mehrere $C_1$-$C_4$-Alkylreste, vorzugsweise Methylgruppen, enthalten und weisen insgesamt 5 bis 10 Kohlenstoffatome auf.

Bevorzugte Substituenten in der Benzylgruppe der R-, T-, X- und Y-Reste, in der Phenylgruppe von $R_1$, $R_2$ und $T_1$ und in der Benzyloxygruppe von X sind z. B. Halogene, Methyl oder Methoxy. Beispiele für derartige araliphatische bzw. aromatische Reste sind p-Methylbenzyl, o- oder p-Chlorbenzyl, o- oder p-Tolyl, Xylyl, o-, m- oder p-Chlorphenyl, o- oder p-Methoxyphenyl, o- oder p-Chlorbenzyloxy oder o- oder p-Methylbenzyloxy.

Wenn die Substituenten $R_1$ und $R_2$ zusammen mit dem gemeinsamen Stickstoffatom einen heterocyclischen Rest darstellen, so ist dieser beispielsweise Pyrrolidino, Piperidino, Pipecolino, Morpholino, Thiomorpholino oder Piperazino z. B. N-Methylpiperazino. Bevorzugte gesättigte heterocyclische Reste für $-NR_1R_2$ sind Pyrrolidino, Piperidino oder Morpholino.

Die Substituenten $R_1$ und $R_2$ sind vorzugsweise Cyclohexyl, Benzyl, Cyano-Niederalkyl z. B. β-Cyanoethyl oder in erster Linie Niederalkyl, wie z. B. Methyl oder vor allem Ethyl. $-NR_1R_2$ ist bevorzugt auch Pyrrolidinyl.

X kann vorteilhafterweise Wasserstoff, Halogen, Niederalkyl, wie z. B. Methyl; Benzyloxy, $C_1$-$C_8$-Alkoxy, in erster Linie Niederalkoxy, wie z. B. Methoxy, Ethoxy, Isopropoxy oder tert. Butoxy, oder die Gruppe $-NT_1T_2$ sein, wobei von den Resten $T_1$ und $T_2$ eines vorzugsweise $C_1$-$C_8$-Acyl oder Niederalkyl und das andere Wasserstoff oder Niederalkyl ist. Der Acylrest ist in diesem Falle besonders Niederalkylcarbonyl, wie z. B. Acetyl oder Propionyl. Vorzugsweise ist X Acetylamino, Dimethylamino, Benzyloxy oder besonders Niederalkoxy und vor allem Ethoxy.

Der N-Substituent Y ist vorzugsweise Benzyl, Acetyl, Propionyl oder besonders Alkyl mit 1 bis 8 Kohlenstoffatomen, wie z. B. Methyl, Ethyl, n-Butyl oder vor allem n-Octyl.

Z ist vorzugsweise Phenyl oder vor allem Methyl.

Die Benzolringe A und B sind vorzugsweise nicht weiter substituiert. Wenn A und B Substituenten enthalten, so sind diese besonders Halogen in Ring A und Niederalkyl z. B. Methyl in Ring B.

Die erfindungsgemässen Azaphthalide stellen vorzugsweise Isomerengemische dar. Diese Gemische weisen in der Regel einen grösseren Teil der 6-Azaphthalidverbindung auf. Das Verhältnis der Isomeren im Gemisch beträgt in der Regel 20 : 1 bis 1,1 : 1, insbesondere 10 : 1 bis 1,5 : 1.

Praktisch wichtige chromogene 5- und 6-Azaphthalide entsprechen der Formel

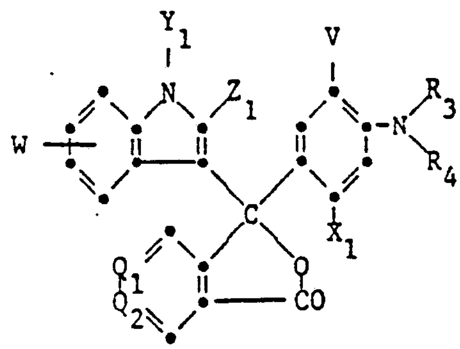
(2)

worin $Q_1$ und $Q_2$ die angegebene Bedeutung haben und

W Halogen oder vorzugsweise Wasserstoff,

$Y_1$ Wasserstoff, Alkyl mit 1 bis 8 Kohlenstoffatomen, Acetyl, Propionyl oder unsubstituiertes oder durch Halogen, Methyl oder Methoxy substituiertes Benzyl,

$Z_1$ Niederalkyl oder Phenyl,

V Wasserstoff, Halogen oder Niederalkyl,

$R_3$ und $R_4$, unabhängig voneinander, Niederalkyl, Cyanoniederalkyl, Cyclohexyl oder unsubstituiertes oder durch Halogen, Methyl oder Methoxy substituiertes Benzyl oder

$R_3$ und $R_4$ zusammen mit dem sie verbindenden Stickstoffatom Pyrrolidino, Piperidino oder Morpholino,

$X_1$ Wasserstoff, Halogen, Niederalkyl, $C_1$-$C_8$-Alkoxy, Benzyloxy oder die Gruppe

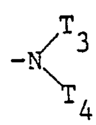
,

$T_3$ und $T_4$, unabhängig voneinander, Wasserstoff, Niederalkyl, Formyl, Niederalkylcarbonyl oder unsubstituiertes oder durch Halogen, Methyl oder Methoxy substituiertes Benzoyl bedeuten.

Halogen in Verbindung mit den vorstehenden Substituenten in Formeln (1) und (2) bedeutet beispielsweise Fluor, Brom oder vorzugsweise Chlor.

Unter den Azaphthaliden der Formel (2), die vorzugsweise in Form von Isomerengemischen vorliegen, sind diejenigen, in denen $X_1$ Niederalkyl, $C_1$-$C_8$-Alkoxy, vorallem Niederalkoxy, Benzyloxy, Formylamino, Niederalkyl carbonylamino, Benzoylamino oder Diniederalkylamino bedeutet, bevorzugt.

$Y_1$ ist vorzugsweise $C_1$-$C_8$-Alkyl und V ist besonders Wasserstoff.

Von besonderem Interresse sind Isomerengemische von Azaphthaliden der Formel

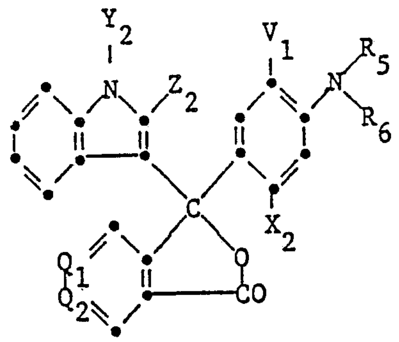

(3)

worin $Q_1$ und $Q_2$ die angegebene Bedeutung haben und

$Y_2$ Wasserstoff, Alkyl mit 1 bis 8 Kohlenstoffatomen, oder Benzyl,

$Z_2$ Phenyl oder vor allem Methyl,

$R_5$ und $R_6$, unabhängig voneinander, je Niederalkyl, Cyclohexyl oder Benzyl oder —$NR_5R_6$ Pyrrolidino, Piperidino oder Morpholino,

$V_1$ Methyl oder vorzugsweise Wasserstoff,

$X_2$ Methyl, Niederalkoxy, Benzyloxy, Acetylamino, Propionylamino, Benzoylamino oder Diniederalkylamino

bedeuten.

Unter diesen Verbindungen der Formel (3) sind diejenigen besonders bevorzugt, bei denen $R_5$ Methyl, Ethyl oder Cyclohexyl, $R_6$ Methyl oder Ethyl oder —$NR_5R_6$ Pyrrolidinyl, $V_1$ Wasserstoff, $X_2$ Niederalkoxy, vor allem Ethoxy oder Acetylamino, $Z_2$ Methyl und $Y_2$ Methyl, Ethyl, n-Butyl oder vor allem n-Octyl bedeuten.

Die erfindungsgemässen Azaphthalide der Formeln (1) bis (3) stellen neue chromogene Verbindungen dar und können nach an sich bekannten Methoden hergestellt werden. Ein Verfahren zur Herstellung der Azaphthalide der Formel (1) besteht darin, dass man eine Verbindung der Formel

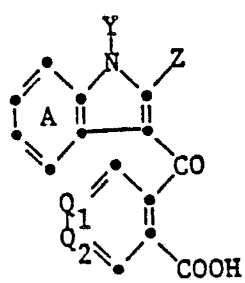

(4)

mit einer Verbindung der Formel

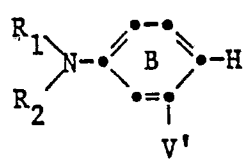

(5)

umsetzt, worin A, B, $Q_1$, $Q_2$, Y, Z, $R_1$ und $R_2$ die angegebene Bedeutung haben und V' die für X angegebene Bedeutung hat oder Hydroxy bedeutet. Stellt V' Hydroxy oder —$NT_1T_2$, worin $T_1$ und/oder $T_2$ Wasserstoff bedeuten, dar, so kann das Reaktionsprodukt nachfolgend noch definitionsgemäss alkyliert bzw. aralkyliert und/oder acyliert werden.

Andererseits können die erfindungsgemässen Azaphthalide auch dadurch hergestellt werden, dass man eine Verbindung der Formel

(Siehe Formel 6 Seite 5 f.)

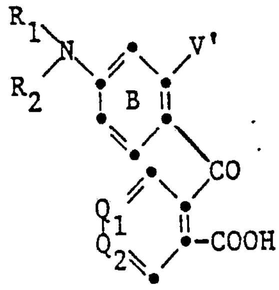

(6)

mit einer Indolverbindung der Formel

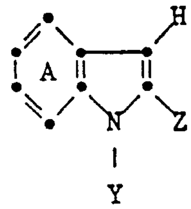

(7)

umsetzt, worin A, B, $Q_1$, $Q_2$, $R_1$, $R_2$, Y und Z die angegebene Bedeutung haben und V' die für X angegebene Bedeutung hat oder Hydroxy bedeutet, und das Reaktionsprodukt noch alkyliert bzw. aralkyliert und/oder acyliert werden kann, wenn V' Hydroxy oder $T_1$ und/oder $T_2$ Wasserstoff bedeuten.

Die Umsetzungen werden vorzugsweise so durchgeführt, dass man die Reaktionskomponenten in Anwesenheit eines sauren Entwässerungsmittels bei einer Temperatur von 20° bis 140 °C zur Reaktion bringt. Beispiele für derartige Kondensationsmittel sind Essigsäureanhydrid, Schwefelsäure, Phosphorsäure und Phosphoroxychlorid.

Die Isolierung des Endproduktes der Formel (1) erfolgt in allgemein bekannter Weise durch Einstellen des Reaktionsgemisches auf einen pH-Wert von mindestens 6, vorzugsweise 7 bis 11, z. B. mit Alkalien wie Alkalimetallhydroxiden, Ammoniak, Alkalimetallcarbonaten oder -bicarbonaten und Abtrennen des gebildeten Niederschlages, Waschen und Trocknen oder durch Behandeln mit geeigneten organischen Lösungsmitteln, wie z. B. Methanol, Isopropanol, Benzol, Chlorbenzol oder vor allem Toluol. Man erhält in der Regel Isomerengemische, aus denen die einzelnen 5- und 6-Azaphthalide durch Chromatographieren und/oder Umkrystallisieren getrennt werden können.

Die Alkylierung oder Aralkylierung bzw. Acylierung der Reaktionsprodukte, bei denen V' Hydroxy oder mindestens eines von $T_1$ und $T_2$ Wasserstoff bedeutet, wird in der Regel nach bekannten Verfahren durchgeführt. Beispielsweise wird die Reaktion in Anwesenheit eines säurebindenden Mittels, wie z. B. eines Alkalicarbonats oder einer tertiären Stickstoffbase, wie z. B. Triethylamin und gegebenenfalls in Anwesenheit eines inerten, organischen Lösungsmittels, wie z. B. Aceton, Isopropylalkohol, Chlorbenzol oder Nitrobenzol, durchgeführt. Geeignete Acylierungsmittel sind z. B. reaktionsfähige, funktionelle Derivate von aliphatischen Carbonsäuren, insbesondere Fettsäurehalogenide und -anhydride, wie z. B. Acetylbromid, Acetylchlorid oder Essigsäureanhydrid oder von aromatischen Carbonsäuren, wie z. B. Benzoesäurehalogenide. Geeignete Alkylierungsmittel sind Alkylhalogenide, wie z. B. Methyl- oder Ethyljodid oder -chlorid oder Dialkylsulfate, wie z. B. Dimethyl- oder Diethylsulfat. Als Aralkylierungsmittel eignet sich insbesondere Benzylchlorid oder die entsprechenden Substitutionsprodukte, wie z. B. p-Chlorbenzylchlorid oder 2,4-Dimethylbenzylchlorid, die vorzugsweise in einem nicht-polaren, organischen Lösungsmittel, wie z. B. Benzol, Toluol oder Xylol, verwendet werden.

Die Ausgangsstoffe der Formeln (4) und (6) werden in der Regel durch Umsetzung des aus Pyridin-3,4-dicarbonsäure erhaltenen Anhydrids der Formel

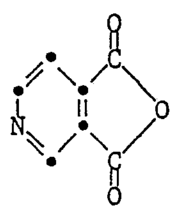

(8)

mit einer Verbindung der Formel (7) bzw. mit einer Verbindung der Formel (5) erhalten, wobei die Reaktion gewünschtenfalls in einem organischen Lösungsmittel und gegebenenfalls in Gegenwart eines organischen oder anorganischen Metallsalzes, z. B. Zinkchlorid oder Aluminiumchlorid, durchgeführt wird. Geeignete organische Lösungsmittel sind z. B. Dimethylformamid, Acetonitril, Propionitril, niedere aliphatische Carbonsäuren, wie z. B. Essigsäure oder Propionsäure, Benzol, Toluol, Xylol oder Chlorbenzol. Die Reaktion erfolgt vorzugsweise bei Temperaturen von 5 °C bis zum Siedepunkt des eingesetzten Lösungsmittels. Die erhaltenen Verbindungen der Formeln (4) und (6) werden vorzugsweise ohne isoliert zu werden zur Umsetzung mit den Anilinverbindungen der Formel (5) bzw. mit den Indolverbindungen der Formel (7) weiterverwendet.

Die Ueberführung der Pyridin-3,4-dicarbonsäure (Cinchomeronsäure) in das Anhydrid erfolgt vorteilhafterweise wie auch für die Chinolinsäure durch Aufheizen der Säure in Essigsäureanhydrid, Eindampfen der Mischung bis zur Trockene und Auflösen oder Suspendieren des trockenen Rückstandes in einer niederen Carbonsäure, wie z. B. Essigsäure, wonach die erhaltene Lösung oder Suspension direkt weiter verwendet werden kann.

Die Verbindungen der Formel (4), worin Y eine gegebenenfalls substituierte Alkyl- oder Benzylgruppe darstellt, können ebenfalls durch Acylierung, Alkylierung bzw. Aralkylierung des Zwischenproduktes nach üblichen Methoden hergestellt werden, das durch Umsetzung des Anhydrids der Formel (8) mit einer Indolverbindung der Formel (7) erhalten wird, worin Y Wasserstoff ist. Die Acylierungs-, Alkylierungs- und Aralkylierungsmittel können die gleichen sein, wie sie für die Herstellung der Verbindungen der Formeln (1) bis (3) angegeben sind.

Die Azaphthalide der Formeln (1) bis (3) sind normalerweise farblos oder höchstens schwach gefärbt. Wenn diese Farbbildner mit einem vorzugsweise sauren Entwickler, d. h. einem Elektronenakzeptor, in Kontakt gebracht werden, so ergeben sie je nach Bedeutung von X und dem verwendeten Entwickler intensive grün-blaue, blaue oder violett-blaue Farbtöne, die ausgezeichnet sublimations- und lichtecht sind. Sie sind deshalb auch sehr wertvoll im Gemisch mit einem oder mehreren anderen bekannten Farbbildner, z. B. 3,3-(Bis-aminophenyl-)-phthaliden, (3,3-Bis-indolyl-)-phthaliden, 3-Indolyl-3-aminophenyl-4-azaphthaliden, 3-Amino-fluoranen, 2,6-Diaminofluoranen, 2,6-Diamino-3-methyl-fluoranen, Leukoauraminen, Spiropyranen, Spirodipyranen, Chromenoindolen, Phenoxazinen, Phenothiazinen, Carbazolylmethanen oder weiteren Triarylmethan-leukofarbstoffen, um blaue, marine-blaue, graue oder schwarze Färbungen zu ergeben.

Die Azaphthalide der Formeln (1) bis (3) zeigen sowohl auf phenolischen Unterlagen, wie auch besonders auf aktivierten Tonen eine ausgezeichnete Farbintensität und Lichtechtheit. Sie eignen sich vor allem als sich schnell entwickelnde Farbbildner für die Verwendung in einem wärmeempfindlichen oder insbesondere druckempfindlichen Aufzeichnungsmaterial, das sowohl Kopier- als auch Registriermaterial sein kann. Sie zeichnen sich dadurch aus, dass sie in den Kapselölen hervorragend löslich sind und durch Belichtung in CB-Blatt eine geringe Abnahme der Farbstärke (CB-Desaktivierung) aufweisen.

Ein druckempfindliches Material besteht beispielsweise aus mindestens einem Paar von Blättern, die mindestens einen Farbbildner der Formeln (1) bis (3) gelöst in einem organischen Lösungsmittel und einen Elektronenakzeptor als Entwickler enthalten.

Typische Beispiele für solche Entwickler sind Aktivton-Substanzen, wie Attapulgus-Ton, Säureton, Bentonit, Montmorillonit, aktivierter Ton, wie z. B. säureaktiviertes Bentonit oder Montmorillonit, ferner Zeolith, Halloysit, Siliciumdioxyd, Aluminiumoxid, Aluminiumsulfat, Aluminiumphosphat, Zinkchlorid, Zinknitrat, aktiviertes Kaolin oder irgendein beliebiger Ton. Als Entwickler können auch sauer reagierende, organische Verbindungen, wie z. B. gegebenenfalls ringsubstituierte Phenole, Resorcine, Salicylsäure oder Salicylsäureester und deren Metallsalze, sowie ein sauer reagierendes, polymeres Material, wie z. B. ein phenolisches Polymerisat, ein Alkylphenolacetylenharz, ein Maleinsäure-Kolophonium-Harz oder ein teilweise oder vollständig hydrolysiertes Polymerisat von Maleinsäureanhydrid mit Styrol, Ethylen oder Vinylmethylether, oder Carboxypolymethylen verwendet werden. Es können auch Mischungen der genannten monomeren und polymeren Verbindungen eingesetzt werden. Besonders bevorzugte Entwickler sind säureaktiviertes Bentonit, Zinksalicylate oder die Kondensationsprodukte von p-substituierten Phenolen mit Formaldehyd. Die letzteren können auch mit Zink modifiziert sein.

Die Entwickler können zusätzlich auch im Gemisch mit an sich unreaktiven oder wenig reaktiven Pigmenten oder weiteren Hilfsstoffen wie Kieselgel oder UV-Absorbern, wie z. B. 2-(2-Hydroxyphenyl-)benzotriazolen eingesetzt werden. Beispiele für solche Pigmente sind : Talk, Titandioxid, Aluminiumoxid, Zinkoxid, Kreide, Tone wie Kaolin, sowie organische Pigmente, z. B. Harnstoff-Formaldehydkondensate (BET-Oberfläche 2-75 $m^2/g$) oder Melamin-Formaldehyd-Kondensationsprodukte.

Der Farbbildner liefert an den Punkten, an denen er mit dem Elektronenakzeptor in Kontakt kommt, eine gefärbte Markierung. Um eine frühzeitige Aktivierung der in dem druckempfindlichen Aufzeichnungsmaterial vorhandenen Farbbildner zu verhindern, werden diese in der Regel von dem Elektronenakzeptor getrennt. Dies kann zweckmässig erzielt werden, indem man die Farbbildner in schaum-, schwamm- oder bienenwabenartigen Strukturen einarbeitet. Vorzugsweise sind die Farbbildner in Mikrokapseln eingeschlossen, die sich in der Regel durch Druck zerbrechen lassen.

Beim Zerbrechen der Kapseln durch Druck, beispielsweise mittels eines Bleistiftes, wird die Farbbildnerlösung auf ein benachbartes mit einem Elektronenakzeptor beschichtetes Blatt übertragen, wodurch eine farbige Stelle erzeugt wird. Die Farbe resultiert aus dem dabei gebildeten Farbstoff, der im sichtbaren Bereich des elektromagnetischen Spektrums absorbiert.

Die Farbbildner werden vorzugsweise in Form von Lösungen in organischen Lösungsmitteln eingekapselt. Beispiele für geeignete Lösungsmittel sind vorzugsweise nichtflüchtige Lösungsmittel, z. B. polyhalogeniertes Paraffin oder Diphenyl, wie Chlorparaffin, Monochlordiphenyl oder Trichlordiphenyl, ferner Tricresylphosphat, Di-n-butylphthalat, Dioctylphthalat, Trichlorbenzol, Trichlorethylphosphat,

aromatische Ether, wie Benzylphenylether, Kohlenwasserstofföle, wie Paraffin oder Kerosin, z. B. mit Isopropyl, Isobutyl, sek-Butyl oder tert.-Butyl alkylierte Derivate von Diphenyl, Naphthalin oder Terphenyl, Dibenzyltoluol, partiell hydriertes Terphenyl, mono- bis tetra-$C_1$-$C_3$-alkylierte Diphenylalkane, benzylierte Xylole, oder weitere chlorierte oder hydrierte, kondensierte, aromatische Kohlenwasserstoffe. Oft werden Mischungen verschiedener Lösungsmittel, insbesondere Mischungen aus Paraffinölen oder Kerosin und Diisopropylnaphthalin oder partiell hydriertem Terphenyl, eingesetzt, um eine optimale Löslichkeit für die Farbbildung, eine rasche und intensive Färbung und eine für die Mikroverkapselung günstige Viskosität zu erreichen.

Die Kapselwände können durch Koazervationskräfte gleichmässig um die Tröpfchen der Farbbildnerlösung herum gebildet werden, wobei das Einkapselungsmaterial z. B. in der US-Patentschrift 2 800 457 beschrieben ist. Die Kapseln können vorzugsweise auch aus einem Aminoplast oder modifizierten Aminoplasten durch Polykondensation gebildet werden, wie es in den britischen Patentschriften 989, 264, 1 156 725, 1 301 052 und 1 355 124 beschrieben ist. Ebenfalls geeignet sind Mikrokapseln, welche durch Grenzflächenpolymerisation gebildet werden, wie z. B. Kapseln aus Polyester, Polycarbonat, Polysulfonamid, Polysulfonat, besonders aber aus Polyamid oder Polyurethan.

Die Farbbildner. der Formeln (1) bis (3) enthaltenden Mikrokapseln können zur Herstellung von druckempfindlichen Kopiermaterialien der verschiedensten bekannten Arten verwendet werden. Die verschiedenen Systeme unterscheiden sich im wesentlichen voneinander durch die Anordnung der Kapseln, der Farbreaktanten und durch das Trägermaterial.

Bevorzugt wird eine Anordnung, bei der der eingekapselte Farbbildner in Form einer Schicht auf der Rückseite eines Uebertragungsblattes und der Elektronenakzeptor in Form einer Schicht auf der Vorderseite eines Empfangsblattes vorhanden sind.

Eine andere Anordnung der Bestandteile besteht darin, dass die den Farbbildner enthaltenden Mikrokapseln und der Entwickler in oder auf dem gleichen Blatt in Form einer oder mehrer Einzelschichten oder in der Papierpulpe vorliegen.

Die Kapseln werden vorzugsweise mittels eines geeigneten Binders auf dem Träger befestigt. Da Papier das bevorzugte Trägermaterial ist, handelt es sich bei diesem Binder hauptsächlich um Papiersbeschichtungsmittel, wie Gummi arabicum, Polyvinylalkohol, Hydroxymethylcellulose, Casein, Methylcellulose, Dextrin, Stärke, Stärkederivate oder Polymerlatices. Letztere sind beispielsweise Butadien-Styrolcopolymerisate oder Acrylhomo- oder -copolymere.

Als Papier werden nicht nur normale Papiere aus Cellulosefasern, sondern auch Papiere, in denen die Cellulosefasern (teilweise oder vollständig) durch Fasern aus synthetischen Polymerisaten ersetzt sind, verwendet.

Die Verbindungen der Formeln (1) bis (3) können auch als Farbbildner in einem thermoreaktiven Aufzeichnungsmaterial verwendet werden. Dieses enthält in der Regel mindestens einen Schichtträger, einen Farbbildner, einen Elektronenakzeptor und gegebenenfalls auch ein Bindemittel und/oder Wachs.

Thermoreaktive Aufzeichnungssysteme umfassen, z. B. wärmeempfindliche Aufzeichnungs- und Kopiermaterialien und -papiere. Diese Systeme werden beispielsweise zum Aufzeichnen von Informationen, z. B. in elektronischen Rechnern, Ferndruckern, Fernschreibern oder in Aufzeichnungsgeräten und Messinstrumenten, wie z. B. Elektrocardiographen, verwendet. Die Bilderzeugung (Markierung) kann auch manuell mit einer erhitzten Feder erfolgen. Eine weitere Einrichtung der Erzeugung von Markierungen mittels Wärme sind Laserstrahlen.

Das thermoreaktive Aufzeichnungsmaterial kann so aufgebaut sein, dass der Farbbildner in einer Bindemittelschicht gelöst oder dispergiert ist und in einer zweiten Schicht der Entwickler in dem Bindemittel gelöst und dispergiert ist. Eine andere Möglichkeit besteht darin, dass sowohl der Farbbildner als auch der Entwickler in einer Schicht dispergiert sind. Das Bindemittel wird in spezifischen Bezirken mittels Wärme erweicht und an diesen Punkten, an denen Wärme angewendet wird, kommt der Farbbildner mit dem Elektronenakzeptor in Kontakt und es entwickelt sich sofort die erwünschte Farbe.

Als Entwickler eignen sich die gleichen Elektronenakzeptoren, wie sie in druckempfindlichen Papieren verwendet werden. Beispiele für Entwickler sind die bereits erwähnten Tonminerale und Phenolharze, oder auch phenolische Verbindungen, wie sie beispielsweise in der DE-PS 1 251 348 beschrieben sind, z. B. 4-tert.-Butylphenol, 4-Phenylphenol, Methylen-bis-(p-phenylphenol), 4-Hydroxydiphenylether, α-Naphthol, β-Naphthol, 4-Hydroxybenzoesäure-methylester oder -benzylester, 4-Hydroxydiphenylsulfon, 4'-Hydroxy-4-methyldiphenylsulfon, 4-Hydroxyacetophenon, 2,2'-Dihydroxydiphenyl, 4,4'-Isopropylidendiphenol, 4,4'-Isopropyliden-bis-(2-methylphenol), 4,4'-Bis-(hydroxyphenyl) valeriansäure, Hydrochinon, pyrogallol, Phloroglucin, p-, m-, o-Hydroxybenzoesäure, Gallussäure, 1-Hydroxy-2-naphthoesäure sowie Borsäure und organische, vorzugsweise aliphatische Dicarbonsäuren, wie z. B. Weinsäure, Oxalsäure, Maleinsäure, Zitronensäure, Citraconsäure und Bernsteinsäure.

Vorzugsweise werden zur Herstellung des thermoreaktiven Aufzeichnungsmaterials schmelzbare, filmbildende Bindemittel verwendet. Diese Bindemittel sind normalerweise wasserlöslich, während die Azaphthalide und der Entwickler in Wasser schwer löslich oder unlöslich sind. Das Bindemittel sollte in der Lage sein, den Farbbildner und den Entwickler bei Raumtemperatur zu dispergieren und zu fixieren.

Bei Einwirkung von Wärme erweicht oder schmilzt das Bindemittel, so dass der Farbbildner mit dem Entwickler in Kontakt kommt und sich eine Farbe bilden kann. Wasserunslösliche oder mindestens in Wasser quellbare Bindemittel sind z. B. hydrophile Polymerisate, wie Polyvinylalkohol, Polyacrylsäure,

Hydroxyethylcellulose, Methylcellulose, Carboxymethylcellulose, Polyacrylamid, Polyvinylpyrrolidon, Gelatine, Stärke oder veretherte Maisstärke.

Wenn der Farbbildner und der Entwickler in zwei getrennten Schichten vorliegen, können in Wasser unlösliche Bindemittel, d. h. in nichtpolaren oder nur schwach polaren Lösungsmitteln lösliche Bindemittel, wie z. B. Naturkautschuk, synthetischer Kautschuk, chlorierter Kautschuk, Alkydharze, Polystyrol, Styrol/Butadien-Mischpolymerisate, Polymethylacrylate, Ethylcellulose, Nitrocellulose und Polyvinylcarbazol, verwendet werden. Die bevorzugte Anordnung ist jedoch diejenige, bei der der Farbbildner und der Entwickler in einer Schicht in einem wasserlöslichen Bindemittel enthalten sind.

Die thermoreaktiven Schichten können weitere Zusätze enthalten. Zur Verbesserung des Weissgrades, zur Erleichterung des Bedruckens der Papiere und zur Verhinderung des Festklebens der erhitzten Feder können diese Schichten, z. B. Talk, Titandioxyd, Zinkoxyd, Aluminiumhydroxyd, Calciumcarbonat (z. B. Kreide), Tone oder auch organische Pigmente, wie z. B. Harnstoff-Formaldehydpolymerisate enthalten. Um zu bewirken, dass nur innerhabl eines begrenzten Temperaturbereiches die Farbe gebildet wird, können Substanzen, wie Harnstoff, Thioharnstoff, Diphenylthioharnstoff, Acetamid, Acetanilid, Stearinsäureamid, Phthalsäureanhydrid, Metallstearate, Phthalsäurenitril, Dimethylterephthalat, oder andere entsprechende, schmelzbare Produkte, welche das gleichzeitige Schmelzen des Farbbildners und des Entwicklers induzieren, zugesetzt werden. Bevorzugt enthalten thermographische Aufzeichnungsmaterialien Wachse, z. B. Carnaubawachs, Montanawachs, Paraffinwachs, Polyethylenwachs, Kondensate höherer Fettsäureamide und Formaldehyde und Kondensate höherer Fettsäuren und Ethylendiamin.

Gegenüber den bisher aus der DE-OS 2 842 263, DE-OS 3 116 815 oder der europäischen Patentpublikation 0082 822 bekannten Isomerengemischen von 4- und 7-Azaphthaliden, zeichnen sich die erfindungsgemässen 5- und 6-Azaphthalide durch den Vorteil aus, dass sie insbesondere in wärmeempfindlichen oder vor allem druckempfindlichen Aufzeichnungssystemen eingesetzt werden können, welche als Farbentwickler einen Lewis-Säure-Typ, wie z. B. Aktivton enthalten.

In den folgenden Beispielen beziehen sich die angegebenen Prozentsätze, wenn nichts anderes angegeben ist, auf das Gewicht.

## Beispiel 1

5,0 g Pyridin-3,4-dicarbonsäure (Cinchomeronsäure) und 5,6 g Essigsäureanhydrid werden während 2 Stunden auf 100 °C geheizt. Hierauf wird die Mischung bis zur Trockene eingedampft und anschliessend mit 20 ml Eisessig aufgenommen. Alsdann fügt man 7,2 g N-Butyl-2-methylindol hinzu. Das Reaktionsgemisch wird 5 Stunden bei Raumtemperatur gerührt, dann mit 20 ml Eisessig, 5,0 g N,N-Diethylamino-phenetol und 5,6 g Essigsäureanhydrid versetzt und weitere 3 Stunden bei 70-80 °C gerührt. Das Reaktionsprodukt wird mit wässeriger Natriumhydroxidlösung alkalisch gestellt und mit Toluol extrahiert. Hierauf wird die Toluolphase abgetrennt, mit Wasser gewaschen, über Natriumsulfat getrocknet und eingedampft. Dadurch erhält man 14 g eines Isomerengemisches in Form eines braunen Oels.

Auf einem mit Säureton beschichteten Papier entwickelt das Isomerengemisch eine blaue Farbe, die ausgezeichnet lichtecht ist.

Durch Chromatographieren von 9,0 g des Isomerengemisches auf eine Kieselgelsäule mit Chloroform/Aceton als Eluiermittel erhält man 5,5 g einer 6-Azaphthalidverbindung der Formel

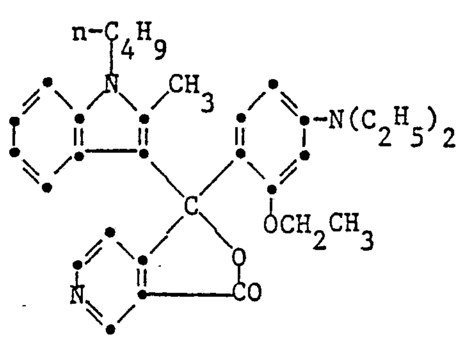

(11)

und 0,9 g einer 5-Azaphthalidverbindung der Formel

(Siehe Formel 12 Seite 9 f.)

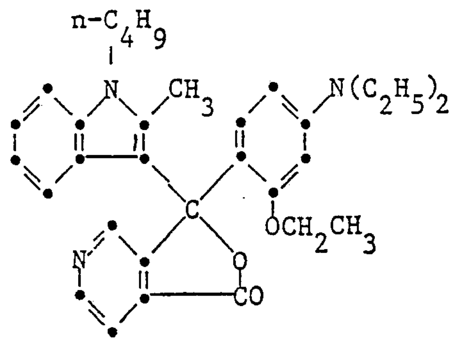

(12)

Nach Umkristallisieren aus Ether schmilzt die 6-Azaphthalidverbindung der Formel (11) bei 131-132 °C, die 5-Azaphthalidverbindung der Formel (12) bei 146-148 °C.

Auf die gleiche Art und Weise wie in Beispiel 1 beschrieben erhält man unter Verwendung der entsprechenden Ausgangsstoffe Isomerengemische, welche nach obengenannter chromatographischer Trennung die in der folgenden Tabelle aufgeführten Azaphthalide der Formel

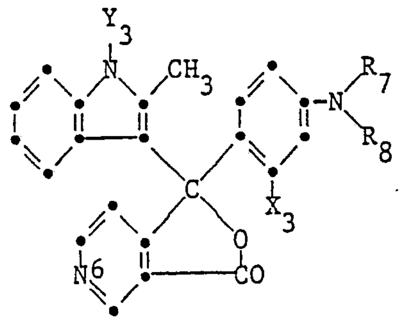

(13)

oder

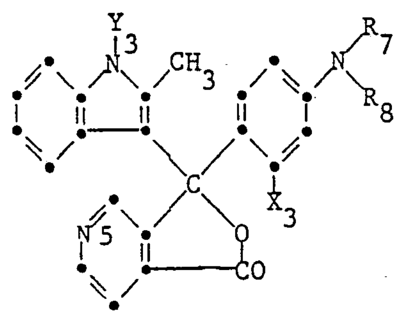

(14)

ergeben.

Tabelle

| Beispiel Nr. | Aza | $Y_3$ | 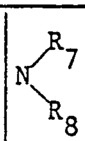 | $X_3$ | Smp. °C | Farbe auf Säureton |
|---|---|---|---|---|---|---|
| 2 | 6-Aza | $-C_2H_5$ | $-N(C_2H_5)_2$ | $-OC_2H_5$ | 182-184 | blau |
| 3 | 5-Aza | $-C_2H_5$ | $-N(C_2H_5)_2$ | $-OC_2H_5$ | 164-166 | blau |
| 4 | 6-Aza | $-n-C_8H_{17}$ | $-N(C_2H_5)_2$ | $-OC_2H_5$ | 118-119 | blau |
| 5 | 5-Aza | $-n-C_8H_{17}$ | $-N(C_2H_5)_2$ | $-OC_2H_5$ | 85-87 | blau |
| 6 | 6-Aza | $-n-C_8H_{17}$ | $-N(C_2H_5)_2$ | $-NHCOCH_3$ | 131-132 | türkis |
| 7 | 6-Aza | $-n-C_8H_{17}$ | 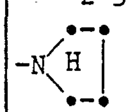 | $-OC_2H_5$ | 158-159 | blau |
| 8 | 6-Aza | $-n-C_8H_{17}$ | $-N(CH_3)_2$ | $-CH_3$ | 151-152 | blau |

Tabelle (Fortsetzung)

| Beispiel Nr. | Aza | $Y_3$ | 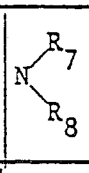 | $X_3$ | Smp. °C | Farbe auf Säureton |
|---|---|---|---|---|---|---|
| 9 | 6-Aza | $-n-C_8H_{17}$ | 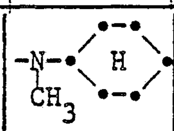 | $-OC_2H_5$ | 78-82 | blau |
| IO | 6-Aza | $-n-C_8H_{17}$ | $-N(C_2H_5)_2$ | 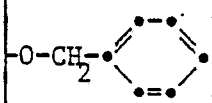 | 129-131 | blau |
| 11 | 6-Aza | H | $-N(C_2H_5)_2$ | $-OC_2H_5$ | 185-187 | blau |
| 12 | 6-Aza | $-CH_3$ | $-N(C_2H_5)_2$ | $-OC_2H_5$ | 205-206 | blau |
| 13 | 6-Aza | H | $-N(C_2H_5)_2$ | $-O-n-C_8H_{17}$ | 80-90 | blau |
| 14 | 6-Aza | $-C_2H_5$ | $-N(C_2H_5)_2$ | $-O-n-C_8H_{17}$ | 107-108 | blau |

Beispiel 15

Herstellung eines druckempfindlichen Kopierpapiers

Eine Lösung von 3 g des Isomerengemisches von den Azaphthaliden der Formeln (11) und (12) (Beispiel 1) in 80 g partiell hydriertem Terphenyl und 17 g Kerosin wird auf an sich bekannte Weise mit Gelatine und Gummi arabicum durch Koazervation mikroverkapselt, mit Stärkelösung vermischt und auf ein Blatt Papier gestrichen. Ein zweites Blatt Papier wird auf der Frontseite mit Säureaktiviertem Bentonit als Farbentwickler beschichtet. Das erste den Farbbildner enthaltende Blatt und das mit Farbentwickler beschichtete Papier werden mit den Beschichtungen benachbart aufeinandergelegt. Durch Schreiben mit der Hand oder mit der Schreibmaschine auf dem ersten Blatt wird Druck ausgeübt, und es entwickelt sich sofort auf dem mit dem Entwickler beschichteten Blatt eine intensive blaue Kopie, die ausgezeichnet sublimier- und lichtecht ist.

Entsprechende intensive, sublimier- und lichtechte blaue bzw. türkise Kopien werden auch bei Verwendung jedes der anderen in den Herstellungs-Beispielen angegebenen Farbbildner der Beispiele 2 bis 14 erzielt.

Beispiel 16

1 g des Isomerengemisches der Azaphthalide gemäss Beispielen 4 und 5 wird in 17 g Toluol gelöst. Zu dieser Lösung gibt man unter Rühren 12 g Polyvinylacetat, 8 g Calciumcarbonat und 2 g Titandioxid. Die erhaltene Suspension wird im Gewichtsverhältnis 1 : 1 mit Toluol verdünnt und mit einem 10 µm Rakel auf ein Blatt Papier gestrichen. Auf dieses Blatt Papier wird ein zweites Blatt Papier gelegt, dessen Unterseite bei einem Auftragsgewicht von 3 g/m² mit einer Mischung bestehend aus 1 Teil eines Amidwachses, 1 Teil eines Stearinwachses und 1 Teil Zinkchlorid beschichtet ist. Durch Schreiben mit der Hand oder mit der Schreibmaschine auf dem oberen Blatt wird Druck ausgeübt, und es entwicklet sich sofort auf dem mit dem Farbbildner beschichteten Blatt eine intensive und lichtechte blaue Farbe.

Beispiel 17

Herstellung eines wärmeempfindlichen Aufzeichnungsmaterials

In einer Kugelmühle werden 32 g 4,4'-Isopropylidendiphenol (Bisphenol A), 3,8 g Distearylamid des Aethylendiamins, 39 g Kaolin, 20 g eines zu 88 % hydrolysierten Polyvinylalkohols und 500 ml Wasser gemahlen bis die Teilchengrösse ca. 5 µm beträgt. In einer zweiten Kugelmühle werden 6 g des Isomerengemisches von den Azaphthaliden gemäss Beispielen 4 und 5, 3 g eines zu 88 % hydrolysierten Polyvinylalkohols und 60 ml Wasser zu einer Teilchengrösse von ca. 3 µm gemahlen.

Die beiden Dispersionen werden zusammengegeben und mit einem Trockenauftragsgewicht von 5,5 g/$m^2$ auf ein Papier gestrichen. Durch Berührung des Papiers mit einem erhitzten Kugelschreiben wird eine intensive blaue Farbe erhalten, die eine ausgezeichnete Licht- und Sublimierechtheit hat.

Intensive und lichtechte blaue bzw. türkise Farben können auch bei Verwendung jedes der anderen Farbbildner gemäss Beispielen 2 bis 14 erhalten werden.

Beispiel 18

In einer Kugelmühle werden 2,7 g des Isomerengemisches aus den Azaphthaliden der Beispiele 4 und 5, 24 g N-Phenyl-N'-(1-hydroxy-2,2,2-trichlorethyl)-harnstoff, 16 g Stearinsäureamid, 59 g eines zu 88 % hydrolysierten Polyvinylalkohols und 58 ml Wasser gemahlen bis die Teilchengrösse 2-5 µm beträgt. Diese Suspension wird bei einem Trockenauftragsgewicht von 5,5 g/$m^2$ auf ein Blatt Papier gestrichen. Durch Berührung des Papiers mit einem erhitzten Kugelschreiber wird eine intensive und lichtechte blaue Farbe erhalten.

**Patentansprüche**

1. 5- und 6-Azaphthalide oder deren Isomerengemische, dadurch gekennzeichnet, dass sie der Formel

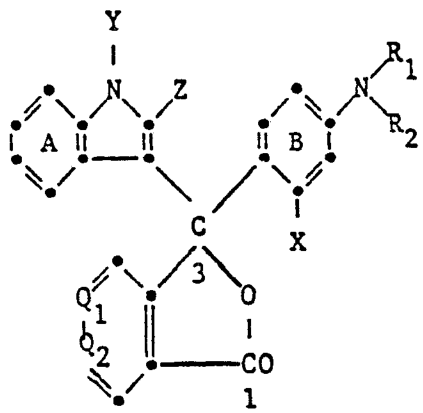

(1)

entsprechen, worin von $Q_1$ und $Q_2$ eines N und das andere CH,

Y Wasserstoff, unsubstituiertes oder durch Halogen, Hydroxyl, Cyano oder $C_1$-$C_5$-Alkoxy substituiertes Alkyl mit höchstens 12 Kohlenstoffatomen, Acyl mit 1 bis 12 Kohlenstoffatomen oder unsubstituiertes oder durch Halogen, Cyano, $C_1$-$C_5$-Alkyl oder $C_1$-$C_5$-Alkoxy substituiertes Benzyl,

Z Wasserstoff, $C_1$-$C_5$-Alkyl oder Phenyl,

$R_1$ und $R_2$ unabhängig voneinander, je Wasserstoff, unsubstituiertes oder durch Halogen, Hydroxyl, Cyano oder $C_1$-$C_5$-Alkoxy substituiertes Alkyl mit höchstens 12 Kohlenstoffatomen, $C_5$-$C_{10}$-Cycloalkyl oder unsubstituiertes oder durch Halogen, Cyano, $C_1$-$C_5$-Alkyl oder $C_1$-$C_5$-Alkoxy substituiertes Benzyl oder Phenyl oder

$R_1$ und $R_2$ zusammen mit dem sie verbindenden Stickstoffatom einen fünf- oder sechsgliedrigen heterocyclischen Rest,

X Wasserstoff, Halogen, $C_1$-$C_5$-Alkyl, $C_1$-$C_{12}$-Alkoxy, $C_1$-$C_{12}$-Acyloxy, Benzyl, Phenyl, Benzyloxy, Phenyloxy, durch Halogen, Cyano, $C_1$-$C_5$-Alkyl oder $C_1$-$C_5$-Alkoxy substituiertes Benzyl oder Benzyloxy, oder die Gruppe

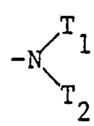 ,

$T_1$ und $T_2$, unabhängig voneinander, je Wasserstoff, $C_1$-$C_5$-Alkyl, $C_5$-$C_{10}$-Cycloalkyl, unsubstituiertes oder durch Halogen, Cyano, $C_1$-$C_5$-Alkyl oder $C_1$-$C_5$-Alkoxy substituiertes Benzyl oder Acyl mit 1 bis 12 Kohlenstoffatomen und $T_1$ auch unsubstituiertes oder durch Halogen, Cyano, $C_1$-$C_5$-Alkyl oder $C_1$-$C_5$-Alkoxy substituiertes Phenyl bedeuten, und

worin der Ring A unsubstituiert oder durch Halogen, Cyano, $C_1$-$C_5$-Alkyl, $C_1$-$C_5$-Alkoxy, $C_1$-$C_5$-Alkoxycarbonyl, Amino, Mono-$C_1$-$C_5$-alkylamino oder Di-$C_1$-$C_5$-alkylamino und der Benzolkern B unsubstituiert oder durch Halogen, $C_1$-$C_5$-Alkyl oder $C_1$-$C_5$-Alkoxy substituiert sind.

2. Azaphthalide gemäss Anspruch 1, dadurch gekennzeichnet, dass in Formel (1) Y $C_1$-$C_8$-Alkyl, Benzyl, Acetyl oder Propionyl bedeutet.

3. Azaphthalide gemäss einem der Ansprüche 1 und 2, dadurch gekennzeichnet, dass in Formel (1) $R_1$ und. $R_2$, unabhängig voneinander, je $C_1$-$C_5$-Alkyl, Cyclohexyl, Benzyl oder Cyano-$C_2$-$C_4$-alkyl bedeuten oder —$NR_1R_2$ Pyrrolidinyl ist.

4. Azaphthalide gemäss einem der Ansprüche 1 bis 3, dadurch gekennzeichnet, dass in Formel (1) X Wasserstoff, Halogen, $C_1$-$C_5$-Alkyl, $C_1$-$C_8$-Alkoxy, Benzyloxy oder die Gruppe —$NT_1T_2$, worin $T_1$ $C_1$-$C_5$-Alkyl oder $C_1$-$C_8$-Acyl und $T_2$ Wasserstoff oder $C_1$-$C_5$-Alkyl sind, bedeutet.

5. Azaphthalide gemäss einem den Ansprüchen 1 bis 4, dadurch gekennzeichnet, dass in Formel (1) die Ringe A und B nicht weitersubstituiert sind.

6. Azaphthalide gemäss Anspruch 1, dadurch gekennzeichnet, dass sie der Formel

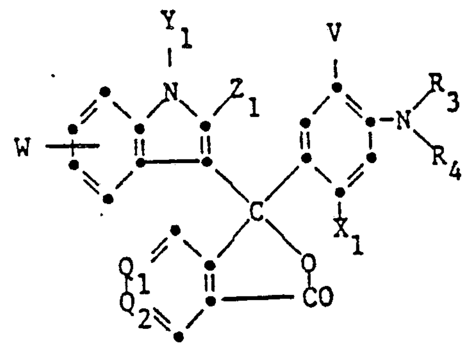

(2)

entsprechen, worin $Q_1$ und $Q_2$ die in Anspruch 1 angegebene Bedeutung haben und

W Wasserstoff oder Halogen,

$Y_1$ Wasserstoff, Alkyl mit 1 bis 8 Kohlenstoffatomen, Acetyl, Propionyl oder unsubstituiertes oder durch Halogen, Methyl oder Methoxy substituiertes Benzyl,

$Z_1$ $C_1$-$C_5$-Alkyl oder Phenyl

V Wasserstoff, Halogen oder $C_1$-$C_5$-Alkyl,

$R_3$ und $R_4$, unabhängig voneinander, je $C_1$-$C_5$-Alkyl, Cyano-$C_2$-$C_4$-alkyl, Cyclohexyl oder unsubstituiertes oder durch Halogen, Methyl oder Methoxy substituiertes Benzyl oder

$R_3$ und $R_4$ zusammen mit dem sie verbindenden Stickstoffatom Pyrrolidino, Piperidino oder Morpholino,

$X_1$ Wasserstoff, Halogen, $C_1$-$C_5$-Alkyl, $C_1$-$C_8$-Alkoxy, Benzyloxy oder die Gruppe

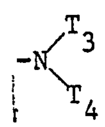,

$T_3$ und $T_4$, unabhängig voneinander, je Wasserstoff, $C_1$-$C_5$-Alkyl, Formyl, $C_1$-$C_5$-Alkylcarbonyl oder unsubstituiertes oder durch Halogen, Methyl oder Methoxy substituiertes Benzoyl bedeuten.

7. Azaphthalide gemäss Anspruch 6, dadurch gekennzeichnet, dass in Formel (2) $X_1$ $C_1$-$C_5$-Alkyl, $C_1$-$C_8$-Alkoxy, Benzyloxy, Formylamino, $C_1$-$C_5$-Alkylcarbonylamino, Benzoylamino oder Di-$C_1$-$C_5$-alkylamino bedeutet.

8. Azaphthalide gemäss Anspruch 6, dadurch gekennzeichnet, dass in Formel (2)

W Wasserstoff,

$Y_1$ Wasserstoff, $C_1$-$C_8$-Alkyl, Acetyl oder Benzyl,

$Z_1$ Methyl oder Phenyl,

$R_3$ und $R_4$, unabhängig voneinander, je $C_1$-$C_5$-Alkyl, Cyclohexyl oder Benzyl oder —$NR_3R_4$ Pyrrolidino, Piperidino oder Morpholino,

V Wasserstoff oder Methyl und

$X_1$ Methyl, $C_1$-$C_5$-Alkoxy, Benzyloxy, Acetylamino, Propionylamino, Benzoylamino oder Di-$C_1$-$C_5$-alkylamino

bedeuten.

9. Azaphthalide gemäss Anspruch 8, dadurch gekennzeichnet, dass

$Y_1$ Methyl, Ethyl, n-Butyl oder n-Octyl,

$Z_1$ Methyl,

$R_3$ Methyl, Ethyl oder Cyclohexyl,

$R_4$ Methyl oder Ethyl oder

—$NR_3R_4$ Pyrrolidino,

V Wasserstoff und

$X_1$ $C_1$-$C_5$-Alkoxy oder Acetylamino

bedeuten.

10. Azaphthalide gemäss einem der Ansprüche 1 bis 9, dadurch gekennzeichnet, dass sie als Isomerengemische vorliegen, in denen das Verhältnis der 6-Aza- zur 5-Azaphthalidverbindung 20 : 1 bis 1,1 : 1, vorzugsweise 10 : 1 bis 1,5 : 1 beträgt.

11. Verfahren zur Herstellung von 5- und 6-Azaphthaliden der im Anspruch 1 angegebenen Formel (1), dadurch gekennzeichnet, dass man eine Verbindung der Formel

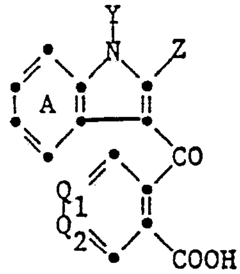

(4)

mit einer Verbindung der Formel

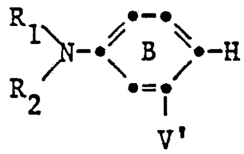

(5)

umsetzt, oder dass man eine Verbindung der Formel

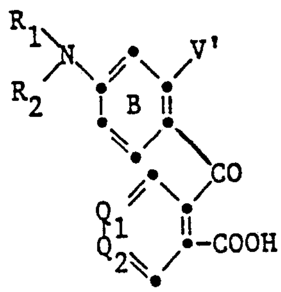

(6)

mit einer Indolverbindung der Formel

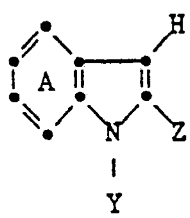

(7)

umsetzt, worin A, B, $Q_1$, $Q_2$, Y, Z, $R_1$ und $R_2$ die in Anspruch 1 angegebene Bedeutung haben und V′ die in Anspruch 1 für X angegebene Bedeutung hat oder Hydroxy bedeutet, und das Reaktionsprodukt noch definitionsgemäss alkyliert, aralkyliert und/oder acyliert, wenn V′ Hydroxy oder $T_1$ und/oder $T_2$ Wasserstoff bedeuten.

12. Verwendung der Azaphthalidverbindungen gemäss einem der Ansprüche 1 bis 10 als Farbbildner in einem druckempfindlichen oder wärmeempfindlichen Aufzeichnungsmaterial.

13. Druckempfindliches Aufzeichnungsmaterial, dadurch gekennzeichnet, dass es in seinem Farbreaktantensystem als Farbbildner Azaphthalidverbindungen gemäss einem der Ansprüche 1 bis 10 und mindestens einen festen Elektronenakzeptor enthält, wobei die Azaphthalidverbindungen, gelöst in einem organischen Lösungsmittel und in Mikrokapseln eingekapselt sind.

14. Druckempfindliches Aufzeichnungsmaterial gemäss Anspruch 13, dadurch gekennzeichnet, dass die eingekapselten Azaphthalidverbindungen in Form einer Schicht auf der Rückseite eines Uebertragungsblattes und der Elektronenakzeptor in Form einer Schicht auf der Vorderseite des Empfangsblattes vorhanden sind.

15. Wärmeempfindliches Aufzeichnungsmaterial, dadurch gekennzeichnet, dass es in mindestens einer Schicht Azaphthalidverbindungen gemäss einem der Ansprüche 1 bis 10, einen Elektronenakzeptor und gegebenenfalls ein Bindemittel und/oder Wachs enthält.

**Claims**

1. A 5- or 6-azaphthalide, or an isomeric mixture thereof, of the formula

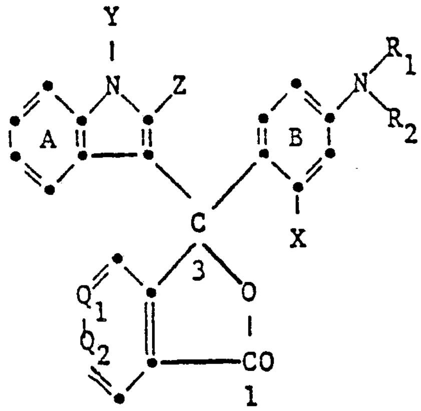

(1)

wherein one of $Q_1$ and $Q_2$ is N and the other is CH,

Y is hydrogen, alkyl having not more than 12 carbon atoms which is unsubstituted or substituted by halogen, hydroxyl, cyano or $C_1$-$C_5$-alkoxy, acyl having 1 to 12 carbon atoms or benzyl which is unsubstituted or substituted by halogen, cyano, $C_1$-$C_5$-alkyl or $C_1$-$C_5$-alkoxy,

Z is hydrogen, $C_1$-$C_5$-alkyl or phenyl,

$R_1$ and $R_2$ are each independently of one another, alkyl having not more than 12 carbon atoms which is unsubstituted or substituted by halogen, hydroxyl, cyano or $C_1$-$C_5$-alkoxy, or are $C_5$-$C_{10}$-cycloalkyl, or benzyl or phenyl, each unsubstituted or substituted by halogen, cyano, $C_1$-$C_5$-alkyl or $C_1$-$C_5$-alkoxy ; or

$R_1$ and $R_2$, together with the nitrogen atom to which they are attached, are a five- or six-membered heterocyclic radical,

X is hydrogen, halogen, $C_1$-$C_5$-alkyl, $C_1$-$C_{12}$-alkoxy, $C_1$-$C_{12}$-acyloxy, benzyl, phenyl, benzyloxy, phenyloxy, or benzyl or benzyloxy, each substituted by halogen, cyano, $C_1$-$C_5$-alkyl or $C_1$-$C_5$-alkoxy, or is the group

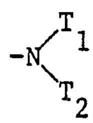

,

in which

$T_1$ and $T_2$ are each independently of one another hydrogen, $C_1$-$C_5$-alkyl, $C_5$-$C_{10}$-cycloalkyl or benzyl which is unsubstituted or substituted by halogen, cyano, $C_1$-$C_5$-alkyl or $C_1$-$C_5$-alkoxy, or are acyl having 1 to 12 carbon atoms, and $T_1$ is also phenyl which is unsubstituted or substituted by halogen, cyano, $C_1$-$C_5$-alkyl or $C_1$-$C_5$-alkoxy.

2. An azaphthalide according to claim 1, wherein in formula (1) Y is $C_1$-$C_8$-alkyl, benzyl, acetyl or propionyl.

3. An azaphthalide according to any one of claims 1 and 2, wherein in formula (1) $R_1$ and $R_2$ are each independently of one another $C_1$-$C_5$-alkyl, cyclohexyl, benzyl or cyano-$C_2$-$C_4$-alkyl or —$NR_1$—$R_2$ is pyrrolidinyl.

4. An azaphthalide according to any one of claims 1 to 3, wherein in formula (1) X is hydrogen, halogen, $C_1$-$C_5$-alkyl, $C_1$-$C_8$-alkoxy, benzyloxy or the —$NT_1T_2$ group, in which $T_1$ is $C_1$-$C_5$-alkyl or $C_1$-$C_8$-acyl and $T_2$ is hydrogen or $C_1$-$C_5$-alkyl.

5. An azaphthalide according to any one of claims 1 to 4, wherein in formula (1) the rings A and B are not further substituted.

6. An azaphthalide according to claim 1, of the formula

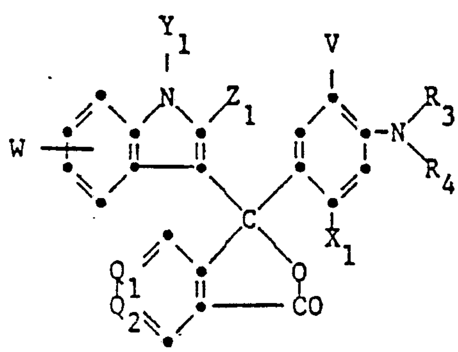

(2)

wherein $Q_1$ and $Q_2$ are as defined in claim 1,

W is hydrogen or halogen,

$Y_1$ is hydrogen, alkyl having 1 to 8 carbon atoms, acetyl, propionyl, or benzyl which is unsubstituted

or substituted by halogen, methyl or methoxy,

$Z_1$ is $C_1$-$C_5$-alkyl or phenyl,

V is hydrogen, halogen or $C_1$-$C_5$-alkyl,

$R_3$ and $R_4$ are each independently of one another $C_1$-$C_5$-alkyl, cyano-$C_2$-$C_4$-alkyl, cyclohexyl, or benzyl which is unsubstituted or substituted by halogen, methyl or methoxy ; or

$R_3$ and $R_4$, together with the nitrogen atom to which they are attached, are pyrrolidino, piperidino or morpholino,

$X_1$ is hydrogen, halogen, $C_1$-$C_5$-alkyl, $C_1$-$C_8$-alkoxy benzyloxy or the group

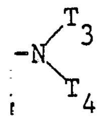 ,

in which

$T_3$ and $T_4$ are each independently of one another hydrogen, $C_1$-$C_5$-alkyl, formyl, $C_1$-$C_5$-alkylcarbonyl, or benzoyl which is unsubstituted or substituted by halogen, methyl or methoxy.

7. An azaphthalide according to claim 6, wherein in formula (2) $X_1$ is $C_1$-$C_5$-alkyl, $C_1$-$C_8$-alkoxy, benzyloxy, formylamino, $C_1$-$C_5$-alkylcarbonylamino, benzoylamino or di-$C_1$-$C_5$-alkylamino.

8. An azaphthalide according to claim 6, wherein in formula (2)

W is hydrogen,

$Y_1$ is hydrogen, $C_1$-$C_8$-alkyl, acetyl or benzyl,

$Z_1$ is methyl or phenyl,

$R_3$ and $R_4$ are each independently of one another $C_1$-$C_5$ alkyl, cyclohexyl or benzyl, or $—NR_3R_4$ is pyrrolidino, piperidino or morpholino,

V is hydrogen or methyl, and

$X_1$ is methyl, $C_1$-$C_5$-alkoxy, benzyloxy, acetylamino, propionylamino, benzoylamino or di-$C_1$-$C_5$-alkylamino.

9. An azaphthalide according to claim 8, wherein

$Y_1$ is methyl, ethyl, n-butyl or n-octyl,

$Z_1$ is methyl,

$R_3$ is methyl, ethyl or cyclohexyl,

$R_4$ is methyl or ethyl, or $—NR_3R_4$ is pyrrolidino,

V is hydrogen and

$X_1$ is $C_1$-$C_5$-alkoxy or acetylamino.

10. An azaphthalide according to any one of claims 1 to 9, which is in the form of an isomeric mixture in which the ratio of 6-azaphthalide compound to 5-azaphthalide compound is 20 : 1 to 1.1 : 1, preferably 10 : 1 to 1.5 : 1.

11. A process for the preparation of a 5- or 6-azaphthalide of the formula (1) as stated in claim 1, which comprises reacting a compound of the formula

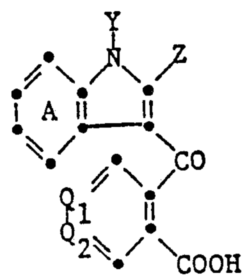

(4)

with a compound of the formula

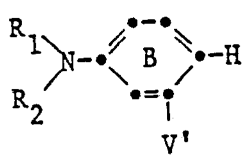

(5)

or reacting a compound of the formula

(See Formula 6 page 16)

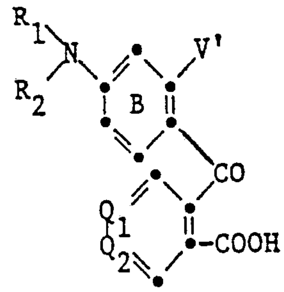

(6)

with an indole compound of the formula

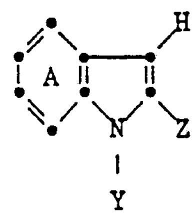

(7)

wherein A, B, $Q_1$, $Q_2$, Y, Z, $R_1$ and $R_2$ have the meanings given in claim 1 and V′ has the meaning given for X in claim 1 or is hydroxyl, and additionally alkylating or aralkylating and/or acylating the reaction product if V′ is hydroxyl or $T_1$ and/or $T_2$ are hydrogen.

12. The use of an azaphthalide according to any one of claims 1 to 10 as colour former in a pressure-sensitive or heat-sensitive recording material.

13. A pressure-sensitive recording material which contains in its colour-reactant system as colour former, azaphthalide compounds according to any one of claims 1 to 10 and at least one solid electron acceptor, wherein the azaphthalide compounds are dissolved in an organic solvent and encapsulated in microcapsules.

14. The pressure-sensitive recording material according to claim 13, wherein the encapsulated azaphthalide compounds are present in the form of a layer on the back of a transfer sheet and the electron acceptor is present in the form of a layer on the face of a receiving sheet.

15. The heat-sensitive recording material which comprises, in at least one layer, azaphthalide compounds according to any one of claims 1 to 10, an electron acceptor and optionally a binder and/or wax.

**Revendications**

1. 5- et 6-azaphtalides ou mélanges d'isomères de ceux-ci, caractérisés par le fait qu'ils correspondent à la formule

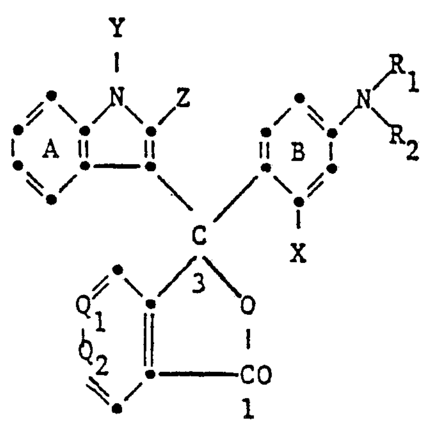

(1)

dans laquelle l'un des chaînons $Q_1$ et $Q_2$ est un N et l'autre CH ;

Y représente un atome d'hydrogène ou un radical alkyle ayant 12 atomes de carbone au maximum, non substitué ou substitué par un atome d'halogène ou par un groupe hydroxyle, cyano ou alcoxy en $C_1$-$C_5$, un radical acyle ayant de 1 à 12 atomes de carbone, ou un radical benzyle non substitué ou substitué par un atome d'halogène ou par un groupe cyano, alkyle en $C_1$-$C_5$ ou alcoxy en $C_1$-$C_5$ ;

Z représente un atome d'hydrogène ou un groupe alkyle en $C_1$-$C_5$ ou phényle ;

$R_1$ et $R_2$ représentent chacun, indépendamment l'un de l'autre, un atome d'hydrogène ou un radical

alkyle ayant 12 atomes de carbone au maximum, non substitué ou substitué par un atome d'halogène ou par un groupe hydroxyle, cyano ou alcoxy en $C_1$-$C_5$, un radical cycloalkyle en $C_5$-$C_{10}$ ou un radical benzyle ou phényle non substitué ou substitué par un atome d'halogène ou par un groupe cyano, alkyle en $C_1$-$C_5$ ou alcoxy en $C_1$-$C_5$ ; ou

$R_1$ et $R_2$ forment, conjointement avec l'atome d'azote auquel ils sont liés, un reste hétérocyclique à 5 ou 6 chaînons ;

X représente un atome d'hydrogène ou d'halogène, ou un radical alkyle en $C_1$-$C_5$, alcoxy en $C_1$-$C_{12}$, acyloxy en $C_1$-$C_{12}$, benzyle, phényle, benzyloxy, phényloxy, un radical benzyle ou benzyloxy substitué par un atome d'halogène ou par un groupe cyano, alkyle en $C_1$-$C_5$ ou alcoxy en $C_1$-$C_5$ ou le groupement

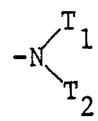 ,

$T_1$ et $T_2$ représentant chacun, indépendamment l'un de l'autre, un atome d'hydrogène ou un groupe alkyle en $C_1$-$C_5$, cycloalkyle en $C_5$-$C_{10}$, un radical acyle ayant de 1 à 12 atomes de carbone ou benzyle non substitué ou substitué par un atome d'halogène ou par un groupe cyano, alkyle en $C_1$-$C_5$ ou alcoxy en $C_1$-$C_5$, et $T_1$ pouvant en outre représenter un radical phényle non substitué ou substitué par un atome d'halogène ou par un groupe cyano, alkyle en $C_1$-$C_5$ ou alcoxy en $C_1$-$C_5$ ; et
dans laquelle le noyau A n'est pas substitué ou est substitué par un atome d'halogène ou par un groupe cyano, alkyle en $C_1$-$C_5$, alcoxy en $C_1$-$C_5$, alcoxy($C_1$-$C_5$)-carbonyle, amino, monoalkyl($C_1$-$C_5$)-amino ou dialkyl-($C_1$-$C_5$)-amino, et le noyau benzénique B n'est pas substitué ou est substitué par un atome d'halogène ou par un groupe alkyle en $C_1$-$C_5$ ou alcoxy en $C_1$-$C_5$.

2. Azaphtalides selon la revendication 1, caractérisés par le fait que dans la formule (1), Y représente un radical alkyle en $C_1$-$C_8$, benzyle, acétyle ou propionyle.

3. Azaphtalides selon l'une des revendications 1 et 2, caractérisés par le fait que dans la formule (1), $R_1$ et $R_2$ représentent chacun, indépendamment l'un de l'autre, un radical alkyle en $C_1$-$C_5$, cyclohexyle, benzyle ou cyanoalkyle($C_2$-$C_4$), ou —$NR_1R_2$ est le groupe pyrrolidinyle.

4. Azaphtalides selon l'une des revendications 1 à 3, caractérisés par le fait que, dans la formule (1), X représente un atome d'hydrogène ou d'halogène, ou un radical alkyle en $C_1$-$C_5$, alcoxy en $C_1$-$C_8$, benzyloxy, ou le groupement —$NT_1T_2$, dans lequel $T_1$ est un groupe alkyle en $C_1$-$C_5$ ou acyle en $C_1$-$C_8$, et $T_2$ est un atome d'hydrogène ou un groupe alkyle en $C_1$-$C_5$.

5. Azaphtalides selon l'une des revendications 1 à 4, caractérisés par le fait que, dans la formule (1), les noyaux A et B ne sont pas substitués davantage.

6. Azaphtalides selon la revendication 1, caractérisés par le fait qu'ils correspondent à la formule

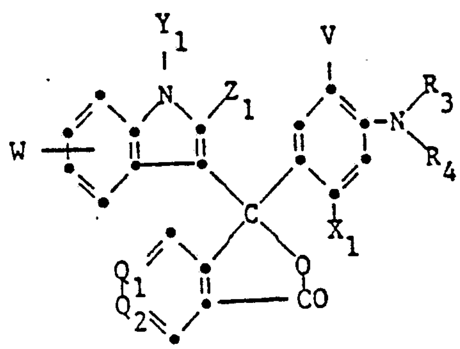 (2)

dans laquelle $Q_1$ et $Q_2$ ont les significations données dans la revendication 1, et

W représente un atome d'hydrogène ou d'halogène ;

$Y_1$ représente un atome d'hydrogène ou un radical alkyle ayant de 1 à 8 atomes de carbone, acétyle, propionyle ou un radical benzyle non substitué ou substitué par un atome d'halogène ou par un groupe méthyle ou méthoxy ;

$Z_1$ représente un groupe alkyle en $C_1$-$C_5$ ou phényle ;

V représente un atome d'hydrogène ou d'halogène, ou un radical alkyle en $C_1$-$C_5$ ;

$R_3$ et $R_4$ représentent chacun, indépendamment l'un de l'autre, un radical alkyle en $C_1$-$C_5$, cyanoalkyle($C_2$-$C_4$), cyclohexyle, ou un radical benzyle non substitué ou substitué par un atome d'halogène ou par un groupe méthyle ou méthoxy, ou

$R_3$ et $R_4$ forment, conjointement avec l'atome d'azote auquel ils sont liés, un reste pyrrolidino, pipéridino ou morpholino ;

$X_1$ représente un atome d'hydrogène ou d'halogène, ou un groupe alkyle en $C_1$-$C_5$, alcoxy en $C_1$-$C_8$, benzyloxy, ou le groupement

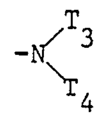 ,

$T_3$ et $T_4$ représentant chacun, indépendamment l'un de l'autre, un atome d'hydrogène ou un radical alkyle en $C_1$-$C_5$, formyle, alkyl($C_1$-$C_5$)-carbonyle, ou un radical benzoyle non substitué ou substitué par un atome d'halogène ou par un groupe méthyle ou méthoxy.

7. Azaphtalides selon la revendication 6, caractérisés par le fait que, dans la formule (2), $X_1$ représente un groupe alkyle en $C_1$-$C_5$, alcoxy en $C_1$-$C_8$, benzyloxy, formylamino, alkyl($C_1$-$C_5$)-carbonylamino, benzoylamino ou dialkyl-($C_1$-$C_5$)-amino.

8. Azaphtalides selon la revendication 6, caractérisés par le fait que, dans la formule (2),

W représente un atome d'hydrogène ;

$Y_1$ représente un atome d'hydrogène ou un groupe alkyle en $C_1$-$C_8$, acétyle ou benzyle ;

$Z_1$ représente le groupe méthyle ou phényle ;

$R_3$ et $R_4$ représentent chacun, indépendamment l'un de l'autre, un groupe alkyle en $C_1$-$C_5$, cyclohexyle ou benzyle, ou —$NR_3R_4$ est le reste pyrrolidino, pipéridino ou morpholino ;

V représente un atome d'hydrogène ou le groupe méthyle ; et

$X_1$ représente un groupe méthyle, alcoxy en $C_1$-$C_5$, benzyloxy, acétylamino, propionylamino, benzoylamino ou dialkyl($C_1$-$C_5$)-amino.

9. Azaphtalides selon la revendication 8, caractérisés par le fait que

$Y_1$ représente le groupe méthyle, éthyle, n-butyle ou n-octyle ;

$Z_1$ représente le groupe méthyle ;

$R_3$ représente le groupe méthyle, éthyle ou cyclohexyle ;

$R_4$ représente le groupe méthyle ou éthyle, ou

—$NR_3R_4$ est le reste pyrrolidino ;

V représente un atome d'hydrogène ; et

$X_1$ représente un groupe alcoxy en $C_1$-$C_5$ ou acétylamino.

10. Azaphtalides selon l'une des revendications 1 à 9, caractérisés par le fait qu'ils se trouvent sous forme de mélanges d'isomères, dans lesquels le rapport du composé 6-aza- au composé 5-azaphtalide va de 20 : 1 à 1,1 : 1, de préférence de 10 : 1 à 1,5 : 1.

11. Procédé pour la préparation de 5- et 6-azaphtalides de formule 1 donnée dans la revendication 1, caractérisé par le fait que l'on fait réagir un composé de formule

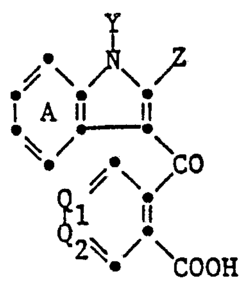

(4)

avec un composé de formule

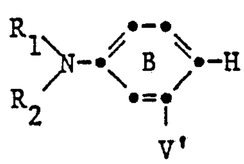

(5)

ou que l'on fait réagir un composé de formule

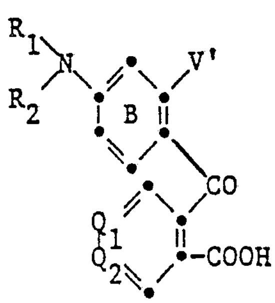

(6)

avec un indole de formule

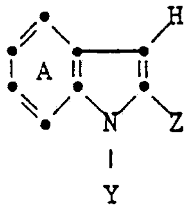

(7)

dans lesquelles formules A, B, $Q_1$, $Q_2$, Y, Z, $R_1$ et $R_2$ ont les significations données dans la revendication 1, et V' a la signification donnée pour X dans la revendication 1 ou représente le groupe hydroxyle, et on soumet en outre le produit de réaction à une alkylation, aralkylation et/ou acylation, conformément aux définitions, lorsque V' représente le groupe hydroxyle ou que $T_1$ et/ou $T_2$ représentent un atome d'hydrogène.

12. Utilisation des composés azaphtalide selon l'une des revendications 1 à 10, en tant que composés chromogènes dans un matériau d'enregistrement sensible à la pression ou sensible à la chaleur.

13. Matériau d'enregistrement sensible à la pression, caractérisé par le fait qu'il contient en tant que composés chromogènes, dans son système de réactifs chromogènes, des composés azaphtalide selon l'une des revendications 1 à 10, et au moins un accepteur d'électrons solides, les composés azaphtalide étant dissous dans un solvant organique et encapsulés dans des microcapsules.

14. Matériau d'enregistrement sensible à la pression selon la revendication 13, caractérisé par le fait que les composés azaphtalide encapsulés sont présents sous forme d'une couche au verso d'une feuille de transfert, et l'accepteur d'électrons est présent sous forme d'une couche au recto de la feuille de réception.

15. Matériau d'enregistrement sensible à la chaleur, caractérisé par le fait qu'il contient, dans au moins une couche, des composés azaphtalide selon l'une des revendications 1 à 10, un accepteur d'électrons et éventuellement un liant et/ou une cire.